(19) Europäisches Patentamt
European Patent Office
Office européen des brevets

(11) Publication number: **0 130 067**
**B1**

(12) **EUROPEAN PATENT SPECIFICATION**

(45) Date of publication of patent specification: **13.09.89**

(51) Int. Cl.$^4$: **C 07 D 209/94**, A 61 K 31/40

(21) Application number: **84304243.3**

(22) Date of filing: **22.06.84**

# (54) Novel indole derivative, processes for its preparation and its use as fertility control agent.

(30) Priority: **23.06.83 GB 8317108**

(43) Date of publication of application:
**02.01.85 Bulletin 85/01**

(45) Publication of the grant of the patent:
**13.09.89 Bulletin 89/37**

(84) Designated Contracting States:
**AT BE CH DE FR GB IT LI LU NL SE**

(56) References cited:

**CHEMICAL ABSTRACTS, vol. 74, no. 25, 21st June 1971, page 133, no. 136409a, Columbus, Ohio, US; B.K.CHOWDHURY et al.: "Chemical taxonomy XXII. 3-Formylindole from Murraya exotica. & PHYTOCHEMISTRY 1971, 10(2), 481-3**

(73) Proprietor: **Kong, Yun Cheung, Dr.**
**A5 Kingston Building, 8th Floor Kingston Street**
**Causeway Bay**
**Hong Kong (HK)**

(72) Inventor: **Kong, Yun Cheung, Dr.**
**A5 Kingston Building, 8th Floor Kingston Street**
**Causeway Bay**
**Hong Kong (HK)**

(74) Representative: **Allam, Peter Clerk et al**
**LLOYD WISE, TREGEAR & CO. Norman House**
**105-109 Strand**
**London WC2R 0AE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 130 067 B1

**Description**

This invention relates to an indole derivative having activity as fertility control agents, to processes for its preparation and to pharmaceutical preparations containing the new compound.

The present compound, hereafter non-systematically named *"Yuehchukene"* was first isolated from *Murraya paniculata* (L.) Jack.

*Murraya paniculata* (L.) Jack is a member of Rutaceae (Citrus Family) widely distributed in tropical and substropical eastern Asia. While wild plants are now found only in isolated pockets in nature, this species is common in horticulture. In taxonomica classification and nomenclature, the scientific name of the species and its synonyms are:

*Murraya paniculata* (L.) Jack, in Malay. Misc. 1:31. 1820. (*sensu lato*).
Synonyms:
*Chalcas paniculata* L., Mant. Pl. 63. 1767.
*Murraya exotica* L., Mant. Pl. 563. 1771.
*Murraya paniculata* var. *exotica* Huang, in Acta Phytotax. Sin. 8:97. 1959.
*Murraya omphalocarpa* Hay., Ic, Pl, Form. 3:51. 1913.
*Murraya paniculata* var. *omphalocarpa* Tan., in J. Soc. Trop. Agr. Form. 1:27, 1929.

Today, it is widely accepted that *Murraya paniculata* and *Murraya exotica* are equivalent taxa; *Murraya paniculata* is described and circumscribed in this sense hereafter.

*Murraya paniculata* has been used for various medicinal purposes. Detailed information on its reputed indications is available in Burkill, I.H. (1935, Dictionary of the Economic Products of the Malay Peninsula. 2 Vols, 2402 pp.), Perry, L. (1980, Medicinal Plants of East and Southeast Asia: Attributed Properties and Uses. MIT Press, Mass. 620 pp.), and Anonymous, (1977, Encyclopaedia of Chinese Materia Medica. Shanhai People's Press 1:1—1489; 2:1491—2754). Indirect references to the use of the species in gynecopathy were mentioned, including amenorrhea, irregular menses, and for labour-induction. In the Encyclopaedia of Chinese Materia Medica just mentioned, it was described under Item 0085: *Jiu Li Xiang gen* (*M. paniculata* root) that an aqueous decoction of this material used as a vaginal suppository could serve as an efficient ecbolic for normal human delivery at term. To the inventor's knowledge, however, no record of its application for anti-implantation or prevention and termination of pregnancy has every been published.

I have now discovered a novel compound in the roots of *M. paniculata* which I have shown possesses anti-implantation activity. The isolation and identification and testing of this new compound, an alkaloid, are described below.

Extensive literature searches have revealed that, so far as is previously known, *M. paniculata* and all other species in its sub-tribe Clauseneae produce no bilogical activity that is related to the regulation of human reproduction. Thus the anti-implantation activity of the present compound is unique.

The present invention is predicated on the isolation from the root of *M. paniculata* of a novel indole derivative whose physical properties are described below and which, from analysis, is believed to have the following structural formula:

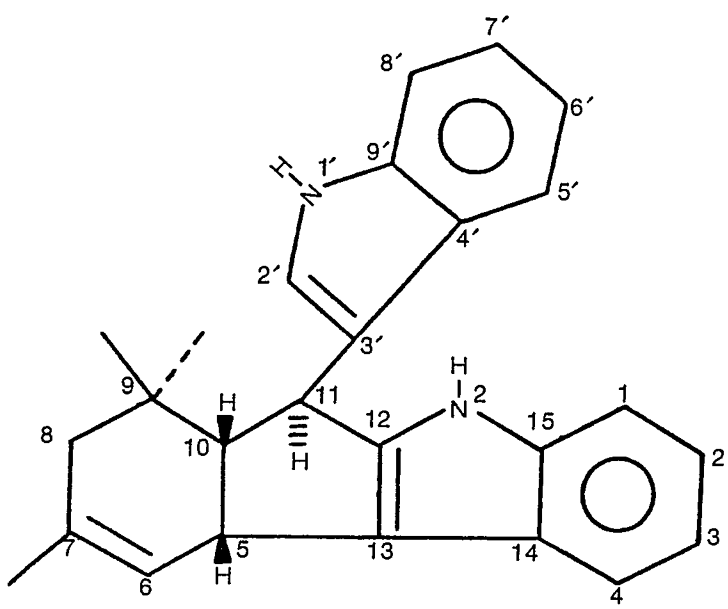

The compound shown by formula I can be described by the systemic name:

11β-(3'-indolyl)-7,9α,9β-trimethyl-5β,8,9,10β-tetrahydro-indano-[2,3-b]-indole, and its mirror image.

In order to commemorate the plant origin of this new compound, it has been given the vernacular name *Yuehchukene*, which, for brevity will be sometimes used in this specification. Reference is made in the following description to the N'-monoacetyl derivative of Yuehchukene, which is not claimed.

Brief Description of the Drawings

Figure 1 shows the two crystalline forms of the N'-monoacetyle derivate of the compound of formula I, i.e. the cubic form and the needle form crystals, this N'-monoacetyl derivative being chosen since its crystal form is amenable to X-ray analysis to enable confirmation of the structure of Yuehchukene;

Figure 2 shows, uteri from rats with those rats to which the compound of formula I has been administered on the right hand side, and those from control rats on the left hand side;

Figure 3*a* shows the uv absorption spectrum of the compound of formula I;

Figure 3*b* shows the ir transmittance of the compound of formula I;

Figure 4 shows the $^1H$ nuclear magnetic resonance spectrum of the compound of formula I in ppm with $CHCl_3$ at 7.25 as reference;

Figure 5 same as above, but showing $D_2O$ exchange in the aromatic region;

Figure 6 shows the $^{13}C$ nmr of the compound of Formula I in ppm with $CDCl_3$ at 76.9 as reference;

Figure 7 shows the mass spectrum of the compound of formula I at 30 electron volt for electronic condensation (eVEI);

Figure 8 shows a structural formula Ia and X-ray picture of the N'-acetyl derivative of the compound of formula I in which R represents N-acetyl group;

Figure 9*a* and 9*b* show the mass spectra of the N'-monoacetyl derivative of the cubic and needle form crystals respectively at 70 eVEI; and

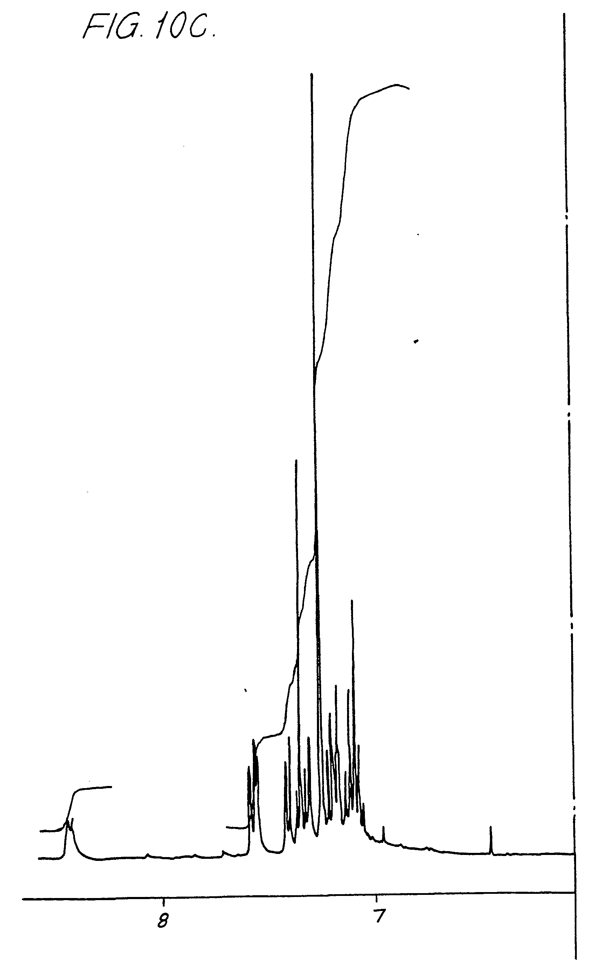

Figure 10 shows the $^1H$ nuclear magnetic resonance spectrum of the N'-monoacetyl derivative (the needle form) in ppm with $CHCl_3$ at 7.25 as reference.

Isolation and Identification of Yuehchukene

Roots harvested from *M. paniculata*, of either domestic or wild origin, were dried in shade. The pulverised material was extracted exhaustively with benzene. The crude extract was concentrated in vacuo at 30°C and the resultant syrup was processed by multiple silica gel chromatography eluted with benzene. At the final stage of purification, Merck Lobar column (Registered Trade Mark) was used with a solvent system of hexane:dichloromethane:isopropanol (9:1:0.5 v/v). The pure compound recovered from the column was a white amorphous powder at very low yield. It can remain in this state at −70°C under nitrogen if it is not further treated in any way, even by dissolving in the same solvent for aliquot distribution. Physical data obtained from this white substance are as follows:

$C_{26}H_{26}N_2$ ($M^+$366.2091, calc. 366.2096)

amorphous white powder, m.p. 127—9°C

λMax EtOH (log ε) 225 nm (4.17), 283 (3.54), 292 (3.44)

$\nu_{KBr}^{cm^{-1}}$ 3402, 2955 2866, 1618, 1548, 1454, 737

n.m.r. $^{13}C\delta ppm$ (90.56 MHz) $^1H\delta ppm$ (360 MHz)

| | | |
|---|---|---|
| C—12 | 130.13 s | |
| C—11/H—11 | 37.54 d | 4.02 dddd |
| C—10/H—10 | 60.80 d | 3.15 ddd |
| C—9 | 33.41 s | — |
| C—8/$H_2$—8 | 41.03 t | 2.28 dd; 1.63 dd |
| C—7 | 136.47 s | — |
| C—6/H—6 | ****120.46 d | 5.70 dq |
| C—5/H—5 | 38.28 d | 4.56 dd |
| C—13 | *120.46 s | — |
| C—14 | **124.23 s | — |
| C—4 | ***118.19 d | 7.43 m |
| C—3 | ****119.44 d | 7.18—7.00 m |

| | | |
|---|---|---|
| C—2 | *****122.08 d | 7.18—7.00 m |
| C—1 | ******111.14 d | *7.35 dd |
| C—15 | *******140.22 s | — |
| C—2′ | 122.90 d | 7.18—7.00 m |
| C—3′ | *118.50 s | — |
| C—4′ | **126.76 s | — |
| C—5′ | ***119.24 d | 7.43 m |
| C—6′ | ****119.47 d | 7.18—7.00 m |
| C—7′ | *****122.16 d | 7.18—7.00 m |
| C—8′ | ******111.60 d | *7.58 dd |
| C—9′ | *******145.09 s | — |
| 9—$Me_2$ | 28.83/28.95 2×q | 1.10/0.86 2 × s |
| 7—Me | 23.91 q | 1.66 broad s |
| | | 7.48 bs, $D_2O$ exchange |
| | | 8.04 bs, $D_2O$ exchange |

Couplings 11/10:8.4; 11/8A: 0.5?; 11/6: 0.5?; 11/5: 1.0; 10/5: 8.4.
8 A/8 B: 17.1

Figure 4 shows the presence and position of hydrogen atoms in the compound and Figure 6 shows the presence and position of carbon atoms in the compound and Figure 5 shows the protons linked to the nitrogen in the indole nuclei

$MS_{(30\ eV)}$: 366($M^+$), 362, 351, 347 (100%), 331, 257, 245, 234, 165, 130, 117.

$MS_{(c.I.)}$: 366 (100%) — see Figure 7

N′-monoacetyl derivative: By refluxing in acetic anhydride in the presence of anhydrous sodium acetate, an N′-monoacetyl derivative with two crystalline forms (see Figure 1) was obtained. Both forms yielded

$MS_{(70\ eV)}$: 408($M^+$), 404, 393, 389, 347 (100%) (see Figures 9*a* and 9*b*).

Pertinent X-ray crystallographic data for the N′-monoacetyl derivatives (the cubic form) is presented in the following table:

Data Collection and Processing Parameters

| | |
|---|---|
| Molecular formula | $C_{28}H_{28}N_2O$ |
| Molecular weight | 408.54 |
| Cell constants | $a$ = 8.032 Å |
| | $b$ = 19.712 |
| | $c$ = 14.460 |
| | $\beta$ = 101.22° |
| | $V$ = 2245.7 $Å^3$ |
| | $Z$ = 4 |
| Density | $\rho_m$ = 1.20 g $cm^{-3}$ |
| | $\rho_c$ = 1.208 g $cm^{-3}$ |

Data Collection and Processing Parameters

| | |
|---|---|
| Space Group | $P2_1/n$ |
| Radiation | graphite-monochromatized Mo*K*α<br>λ = 0.71069 Å |
| Absorption coefficient | 0.68 $cm^{-1}$ |
| Scan type and speed | ω—2θ; 2.02—8.37 deg $min^{-1}$ |
| Scan range | 1° below $K\alpha_1$ to 1° above $K\alpha_2$ |
| Background counting | stationary counts for one-half of scan time at each end of scan |
| Collection range | *h, k,* ±l; $2\theta_{max}$ = 40° |
| Unique data measured | 1876 |
| Observed data with | |
| $(F\| > 3\sigma(\|F\|), n$ | 1523 |
| Number of variables, *p* | 167 |
| $R = \Sigma\|\|F_o\| - \|F_c\|\|/\Sigma\|F_o\|$ | 0.071 |
| Weighting scheme | $w = [\sigma^2(F) + 0.001\|F\|^2]^{-1}$ |
| $R_w = [\Sigma w(\|F_o\| - \|F_c\|)^2/\Sigma w\|F_o\|^2]^{\frac{1}{2}}$ | 0.084 |
| $S = [\Sigma w(\|F_o\| - \|F_c\|)^2/(n-p)]^{\frac{1}{2}}$ | 1.901 |

These data supported the racemic nature of the parent compound and confirmed the nmr assignments for carbon and proton.

The pmr signals of the N'-monoacetyl derivative (the needle form), which has no optical activity, m.p. 243—5°C (decomp), are identical with the parent compound except for a down-field shift of H—2' and H—7' due to the presence of the acetyl group (see Figure 10). This is good evidence that no re-arrangement has occurred during the acetylation process.

Since both the X-ray data of the cubic form crystal and the pmr data of the needle form crystal are agreeable with the structure of the parent compound, and the mass spectra of the two crystalline forms are identical, it is fair to say that the two crystal forms are actually referring to the same compound with a different orientation of the acetyl group. Such crystal dimorphism is not uncommon. The N-acetyl derivative of oxo-butenyl-3-indole also exists in two forms, viz. the needle and the cubic forms, exactly like the crystal in question.

To conclude, all physical data support the structure of formula I above.

Another procedure by which the compound of formula I can be isolated is as follows:

Pulverised *M. paniculata* root material was extracted exhaustively with chloroform at room temperature. The total volume of the extract was concentrated under partial vacuum at 35°C until a syrup was obtained. This is the crude extract. When the crude extract was put on a silica gel column (sample to silica gel 1:10 w/w) and eluted with benzene to methanol and monitored by thin layer chromatography (TLC), only one early fraction with one main component eluted by benzene corresponded to the active compound. Further purification on silica gel column using the TLC solvent system (*vide infra*) yielded the pure compound. Although several known compounds were isolated in this exercise, they are not active. Thus *M. paniculata* root is monovalent in terms of the chemical basis of its anti-implantation activity. As to other plant parts, such activity is not found in extracts from the leaves and stem bark even when the highest tolerable doses were administered in rats.

Fractionation of the crude extract monitored by bioassay leads to the isolation of a pure active compound with the physical properties stated above.

Testing of material extracted from *M. paniculata*

As described in more detail below, the total chloroform extract of *M. paniculata* root material can prevent implantation when primiparous female rats mated by proven males were dosed orally with this crude extract at pregnancy Day 1 to Day 10 (hereinafter as $PD_{1-10}$). This observation holds true when similar

rats were dosed on $PD_{1-2}$ only. The same experimental porotocol carried out in three other laboratories round the world (dosing $PD_{1-10}$), using the same material supplied under code yielded positive results. A negative control containing the debarked stem yielded negative results. This exercise proves *de facto* the genuine anti-implantation activity in *M. paniculata* root. The non-toxic nature of the crude extract is evident since 5 times the effective dose (delivered in $PD_{1-2}$) is not harmful to the animals. Similar experiments with the total alcohol extract produced similar results. The aqueous decoction, similarly tested, was ineffective.

A semi-purified extract, containing the active compound, and dosed at 1.2 g crude extract/Kg in primiparous hamsters mated with proven males on $PD_{1-8}$ yielded positive results (control group 9 pregnant over 10 dosed; experimental group 5 pregnant over 10 dosed). This indicates the non-estrogenic nature of the anti-implantation compound shown active in rat. Indeed, after having autopsied a few hundred female rats treated with crude extracts or pure compounds from *M. paniculata*, not a single animal with signs of estrogenic action in the female genital tract was observed.

The following provides the data on which the above observations are made in greater detail.

Tests for Anti-implantation effect and absence of estrogenic effect of the pure compound (Yuehchukene)

1. Rats (mated, primiparous) were dosed orally at 2.5 mg/Kg on $PD_{1-2}$ and autopsied on $PD_8$. It was 100% active; control 5 pregnant over 5 dosed, experimental zero over 6 dosed.

2. Rats (mated, primiparous) dosed at 2.0 mg/Kg on $PD_{1-6}$ and autopsied on $PD_{16}$ control 10/10, experimental 0/10, 100% active — see Figure 2 from which it can be observed that the uteri from the control rats (left-hand side of Figure 2) show, in every case, implanted foeti whereas the uteri from the rats dosed with the compound of the present invention show no implanted foeti.

3. Rats (mated, primiparous) were dosed orally at 2.5 mg/Kg on $PD_{3-4}$ and autopsied on $PD_8$; control 6/6; experimental 1/6. Dosing after $PD_4$ was inactive.

4. Immature female rats 21 days old, were ovariectomised. At 28 days old, they were dosed orally at 7.5 mg/Kg twice daily for 3 days then autopsied on the fourth day, (n = 7). There was no change in uterine weight, with or without fluid, as compared to control (n = 5), whereas estrogen at the same dose level showed a significant increase in uterine weight with fluid and a slight increase in uterine weight without fluid (n = 7).

5. Hamsters (mated, primiparous) were dosed at 7.5 mg/kg for $PD_{1-8}$ and autopsied on $PD_{14}$. Control group: 6 pregnant over 8 dosed; experimental group: 4 pregnant over 9 dosed.

6. Studies in zygote transport in rats provided further evidence for the absence of estrogenicity. Rats were dosed on $PD_{1-2}$ at 2.5 mg/kg and autopsied on $PD_3$ or $PD_4$. As compared with the corresponding controls, rats treated with *Yuehchukene* and autopsied on $PD_3$ showed 100% pregnancy but only 66% of the total number of zygotes were recovered. They were mainly found in the oviduct. Under similar conditions and at 0.6 mg/kg, 17-beta-ethynylestradiol caused a near-total disappearance of zygotes. Only one zygote was found in the oviduct of one rat. Autopsy on $PD_4$ revealed that 34% of the zygotes were found in the uterus. Under similar conditions, 17-beta-ethynylestradiol caused a total disappearance of the zygotes from the oviduct and the uterus.

It is thus demonstrated that *Yuehchukene* possesses activity as an anti-implantation agent.

Synthesis of *Yuehchukene*

I have discovered that *Yuehchukene* may be synthesized by the Diels-Alder condensation of 3-(3-methyl-1,3-butadienyl)idole. This provides therefore an alternative route for the preparation of *Yuehchukene*.

The essential steps of the synthesis route which I have developed are exemplified in the following example:

Step 1

Synthesis of oxobutenylindole (OBI)

To a suspension of 30 g 3-indolecarboxyaldehyde in 172 ml methanol and 76 ml acetone, 60% KOH in water was added dropwise to a final volume of 207 ml. The suspension was constantly stirred and maintained in an ice-bath. After adding KOH solution, the whole mixture, always stirred, was allowed to rise slowly to room temperature and maintained stirred for 5 days. At the end of this Aldol condensation, glacial acetic acid was added until the reaction mixture turned acidic. The precipitate was collected and taken up in diethyl ether. OBI was obtained by separation on silica gel column with 7% acetic acid in chloroform as eluant. OBI fractions were pooled and allowed to recrystallise in ethanol. A yellow crystal, m.p. 141—2°C, was obtained at 25% yield. The structure was confirmed by ir, uv, ms and pmr.

Step 2

Synthesis of methylbutadienylindole (MBDI)

To a suspension of 10 g methyltriphenylphosphonium bromide in 25 ml freshly distilled tetrahydrofuran (THF), was added dropwise 20 ml of 15% butyllithium in hexane, under nitrogen with constant stirring. The stirring was maintained for 3 hours at room temperature after which a solution of 2.5 g OBI in THF was added dropwise. The stirring was maintained for another 1.5 hour at room temperature, then the mixture was refluxed for 2 hours. At the end of this reaction, water was added and the mixture was

partitioned with diethyl ether. The ether phase was dehydrated with anhydrous magnesium sulphate and evaporated to dryness. The residue was taken up in benzene and loaded on a silica gel column. The column was eluted with benzene and fractions containing MBDI were pooled. MBDI was confirmed by ir, uv, ms and pmr.

Synthesis of *Yuehchukene*

100 mg of MBDI was dissolved in benzene and 5 g of silica gel was added. The slurry was constantly stirred for 6—12 hours. At the end of reaction, the slurry was packed into a small column. Benzene was used to rinse the column and fractions containing *Yuehchukene* were pooled. It compares favourably with authentic *Yuehchukene* from natural sources in silica gel TLC developed by several solvent systems. The $R_f$ and the colour reaction after $H_2SO_4$ spray and heating is the same for the reference and the synthetic compound. The synthetic *Yuehchukene* was also identified by pmr.

Pharmaceutical Preparations

The active compound of the present invention can be formulated with conventional pharmaceutically acceptable carriers and diluents for administration to human beings or other warm-blooded animals for the prevention of pregnancy. The preferred dosage regimen is from 1 to 5 mg per Kg body weight of the active compound, to be taken in 4 consecutive daily dose, the first of which s to be taken 48 hours post coitum. This corresponds to a unit dosage form containing from 50—250 mg of the active implantation agent. The most preferred dosage is about 2.5 mg/Kg.

Examples of suitable pharmaceutical preparations for administration to human beings are as follows:

(a) *Yuehchukene* at 10% dissolved in edible oil base, e.g. olive oil, and stabilised with an anti-oxidant, for oral administration.

(b) *Yuehchukene* in micronised state, suspended in a physiological buffer, for oral administration, or i.m. injection.

(c) a water-soluble derivative of *Yuehchukene* in vials for administration *per os* or *per injectio*.

(d) *Yuehchukene per se* in oil form encapsuled in an edible cover such as gelatin, for oral administration.

(e) *Yuehchukene per se* in powder form, incorporated in a 50% filler base, e.g. starch, as a compressed tablet, for oral administration.

**Claims for the Contracting States: BE CH DE FR GB IT LI LU NL SE**

1. The compound 11β-(3′-indolyl)-7,9α,9β-trimethyl-5β,8,9,10β-tetrahydroindano-[2,3-b]-indole, or a stereo isomer thereof.

2. A process for obtaining 11β-(3′-indolyl)-7,9α,9β-trimethyl-5β,8,9,10β,-tetrahydroindano-[2,3-b]-indole, characterized by subjecting root of *Murraya Paniculata* to solvent extraction with an organic solvent.

3. A process for preparing 11β-(3′-indolyl)-7,9α,9β-trimethyl-5β,8,9,10β-tetrahydroindano-[2,3-b]-indole, characterized by subjecting 3-(3-methyl-1,3-butadienyl)indole to a Diels-Alder condensation reaction.

4. A pharmaceutical preparation, comprising a compound according to Claim 1, and a pharmaceutically acceptable carrier or diluent.

5. The compound 11β-(3′-indolyl)-7,9α,9β-trimethyl-5β,8,9,10β,-tetrahydroindano-[2,3-b]-indole for use as an anti-implantation agent.

**Claims for the Contracting State: AT**

1. A process for obtaining 11β-(3′-indolyl)-7,9α,9β-trimethyl-5β,8,9,10β,-tetrahydroindano-[2,3-b]-indole, characterized by subjecting root of *Murraya paniculata* to solvent extraction with an organic solvent.

2. A process for preparing 11β-(3′-indolyl)-7,9α,9β-trimethyl-5β,8,9,10β-tetrahydroindano-[2,3-b]-indole, characterized by subjecting 3-(3-methyl-1,3-butadienyl)indole to a Diels-Alder condensation reaction.

**Patentansprüche für die Vertragsstaaten: BE CH DE FR GB IT LI LU NL SE**

1. Die Verbindung 11β-(3′-Indolyl)-7,9α,9β-trimethyl-5β,8,9,10β-tetrahydroindano-[2,3-b]-indol, oder eines ihrer Stereoisomeren.

2. Verfahren zur Gewinnung von 11β-(3′-Indolyl)-7,9α,9β-trimethyl-5β,8,9,10β-tetrahydroindano-[2,3-b]-indol, dadurch gekennzeichnet, daß man die Wurzel von *Murraya paniculata* einer Lösungsmittelextraktion mittels eines organischen Lösungsmittels unterzieht.

3. Verfahren zur Herstellung von 11β-(3′-Indolyl)-7,9α,9β-trimethyl-5β,8,9,10β-tetrahydroindano-[2,3-b]-indol, dadurch gekennzeichnet, daß man 3-(3-Methyl-1,3-butadienyl)indol einer Diels-Alder-Kondensation unterzieht.

4. Arzneimittelpräparation, enthaltend eine Verbindung nach Anspruch 1, sowie einen pharmazeutisch annehmbaren Träger oder Verdünnungsmittel.

5. Die Verbindung 11β-(3'-Indolyl)-7,9α,9β-trimethyl-5β,8,9,10β-tetrahydroindano-[2,3-b]-indol zur Verwendung als Antiimplanatationsmittel.

**Patentansprüche für den Vertragsstaat: AT**

1. Verfahren zur Gewinnung von 11β-(3'-Indolyl)-7,9α,9β-trimethyl-5β,8,9,10β-tetrahydroindano-[2,3-b]-indol, dadurch gekennzeichnet, daß man die Wurzel von *Murraya paniculata* einer Lösungsmittelextraktion mittels eines organischen Lösungsmittels unterzieht.

2. Verfahren zur Herstellung von 11β-(3'-Indolyl)-7,9α,9β-trimethyl-5β,8,9,10β-tetrahydroindano-[2,3-b]-indol, dadurch gekennzeichnet, daß man 3-(3-Methyl-1,3-butadienyl)indol einer Diels-Alder-Kondensation unterzieht.

**Revendications pour les Etats contractants: BE CH DE FR GB IT LI LU NL SE**

1. Le composé (indolyl-3')-11β-triméthyl-7,9α,9β-tétrahydro-5β,8,9,10β-indano-indole-[2-3-b], ou un stéréoisomère de celui-ci.

2. Un procédé d'obtention de l'(indolyl-3')-11β-triméthyl-7,9α,9β-tétrahydro-5β,8,9,10β-indano-indole-[2-3-b], caractérisé par la soumission de racine de *Murraya Paniculata* à une extraction par un solvant à l'aide d'un solvant organique.

3. Un procédé d'obtention de l'(indolyl-3')-11β-triméthyl-7,9α,9β-tétrahydro-5β,8,9,10β-indano-indole-[2-3-b], caractérisé par la soumission de (méthyl-3-butadiényl-1,3)-3 indole à une réaction de condensation de Diels-Alder.

4. Une préparation pharmaceutique comprenant un composé selon la revendication 1 et un diluant ou un support pharmaceutiquement acceptable.

5. Le composé (indolyl-3')-11β-triméthyl-7,9α,9β-tétrahydro-5β,8,9,10β-indano-indole-[2-3-b] en vue de son emploi en tant qu'agent anti-implantation.

**Revendications pour l'Etat contractant: AT**

1. Un procédé d'obtention de l'(indolyl-3')-11β-triméthyl-7,9α,9β-tétrahydro-5β,8,9,10β-indano-indole-[2-3-b], caractérisé par la soumission de racine de *Murraya Paniculata* à une extraction par un solvant à l'aide d'un solvant organique.

2. Un procédé d'obtention de l'(indolyl-3')-11β-triméthyl-7,9α,9β-tétrahydro-5β,8,9,10β-indano-indole-[2-3-b], caractérisé par la soumission de (méthyl-2-butadiényl-1,3)-3 indole à une réaction de condensation de Diels-Alder.

FIG. 1.

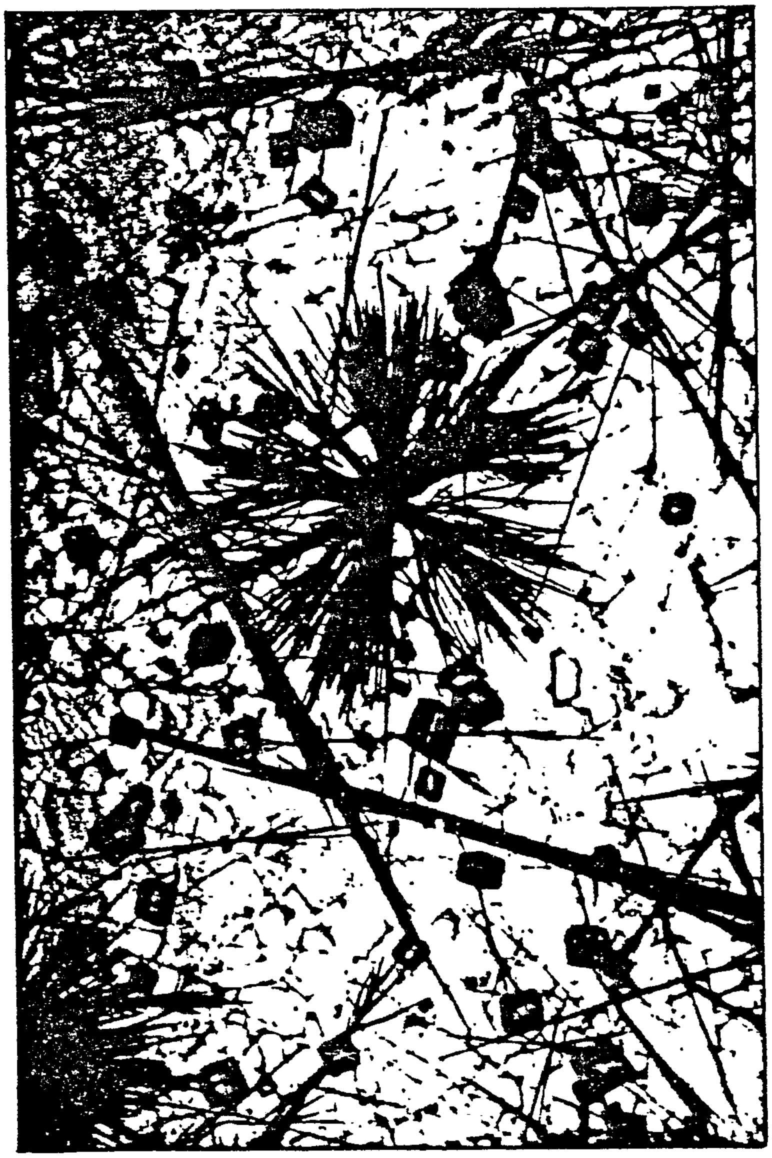

FIG. 2.

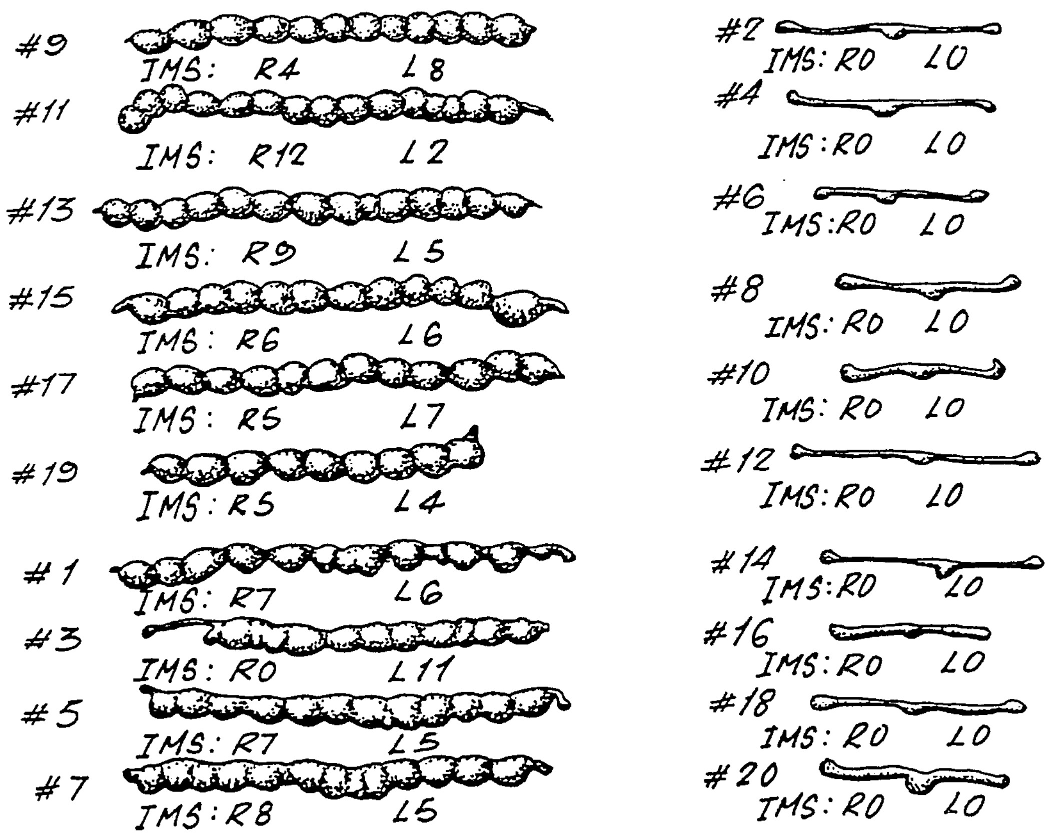
#9
IMS: R4 L8
#11
IMS: R12 L2
#13
IMS: R9 L5
#15
IMS: R6 L6
#17
IMS: R5 L7
#19
IMS: R5 L4
#1
IMS: R7 L6
#3
IMS: R0 L11
#5
IMS: R7 L5
#7
IMS: R8 L5
#2
IMS: R0 L0
#4
IMS: R0 L0
#6
IMS: R0 L0
#8
IMS: R0 L0
#10
IMS: R0 L0
#12
IMS: R0 L0
#14
IMS: R0 L0
#16
IMS: R0 L0
#18
IMS: R0 L0
#20
IMS: R0 L0

FIG. 3a.

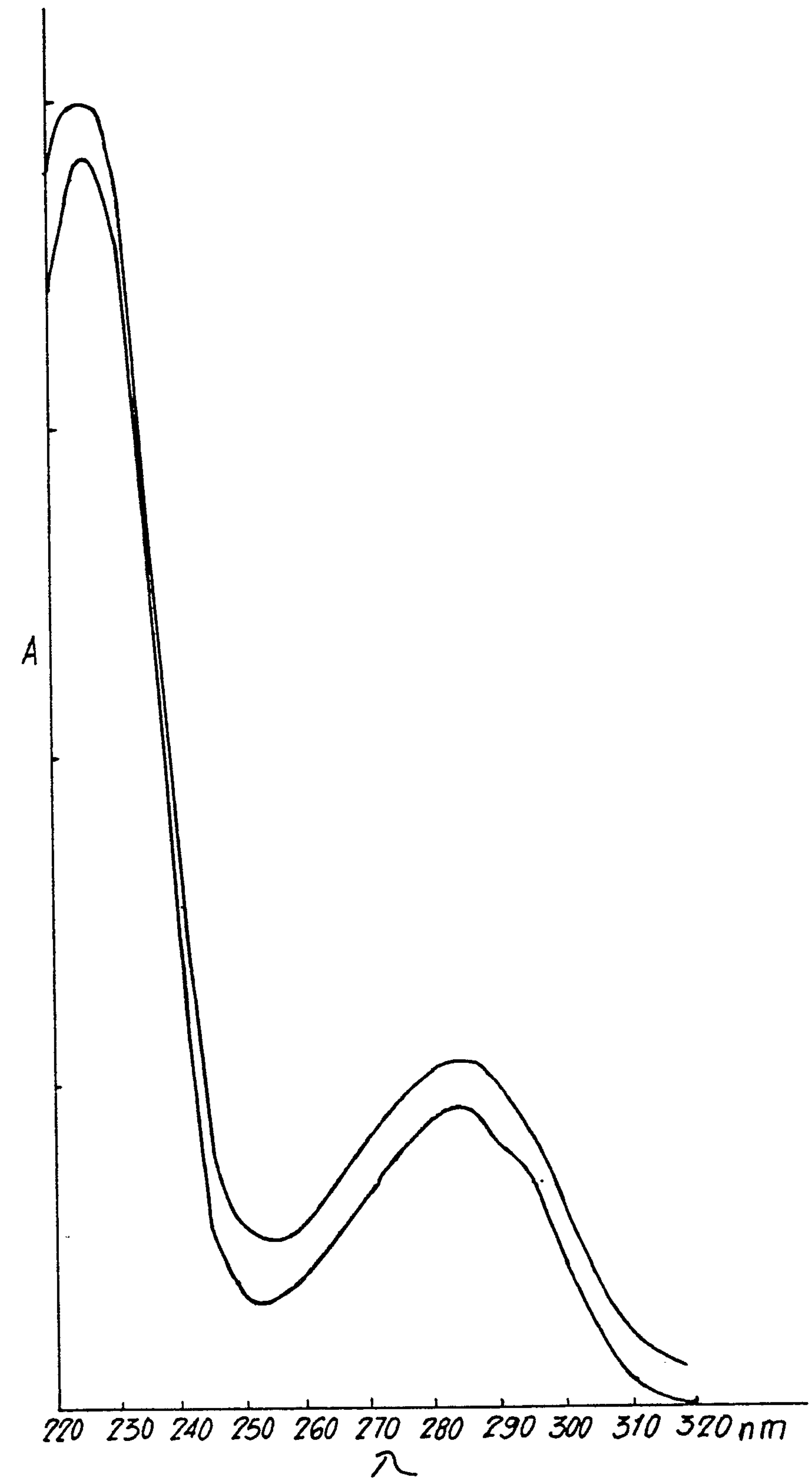

FIG. 3b.

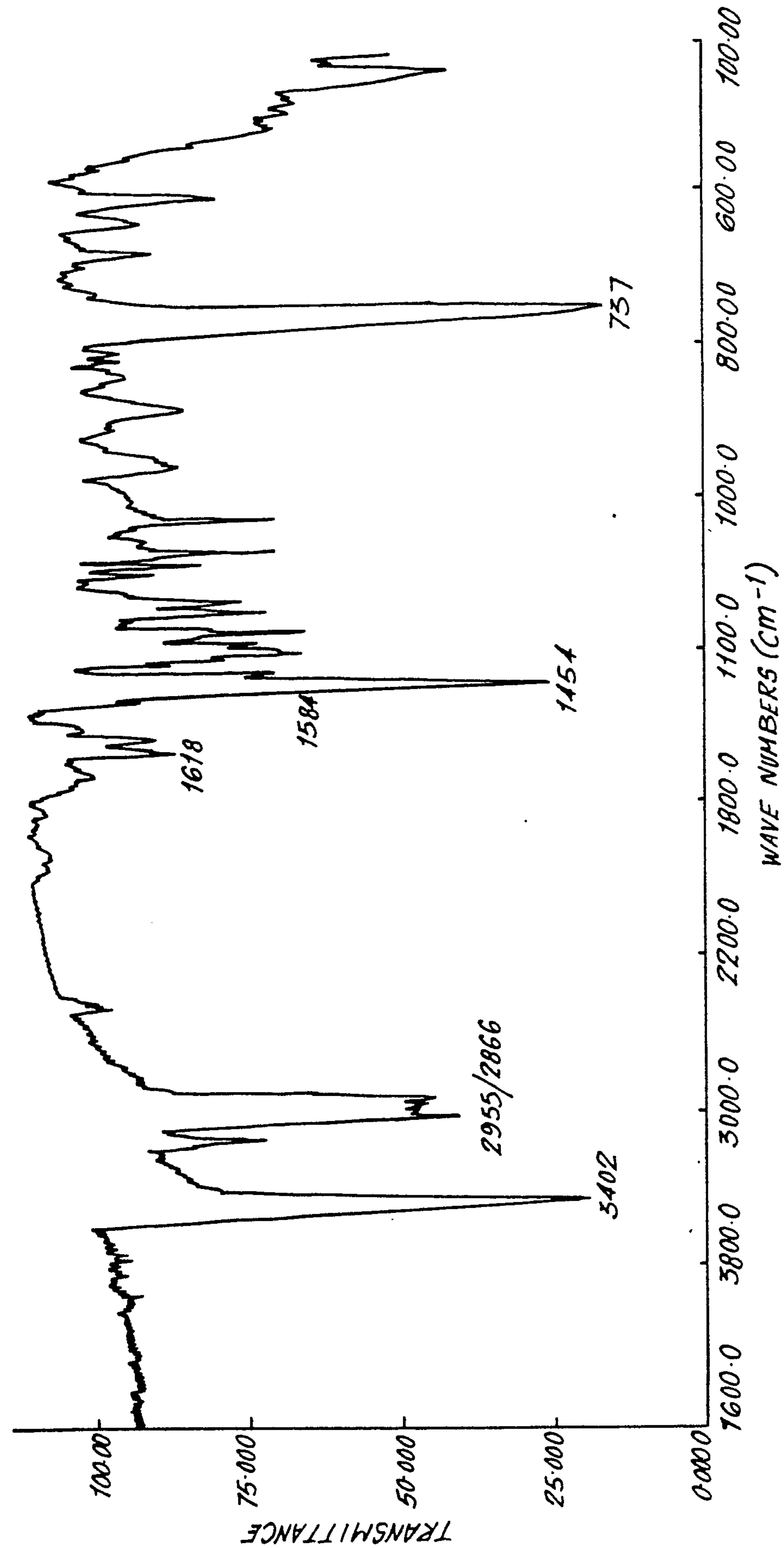

TRANSMITTANCE
100.00
75.000
50.000
25.000
0.0000
WAVE NUMBERS (cm⁻¹)
4600.0
3800.0
3000.0
2200.0
1800.0
1100.0
1000.0
800.00
600.00
100.00
3402
2955/2866
1618
1584
1454
757

FIG. 4a.

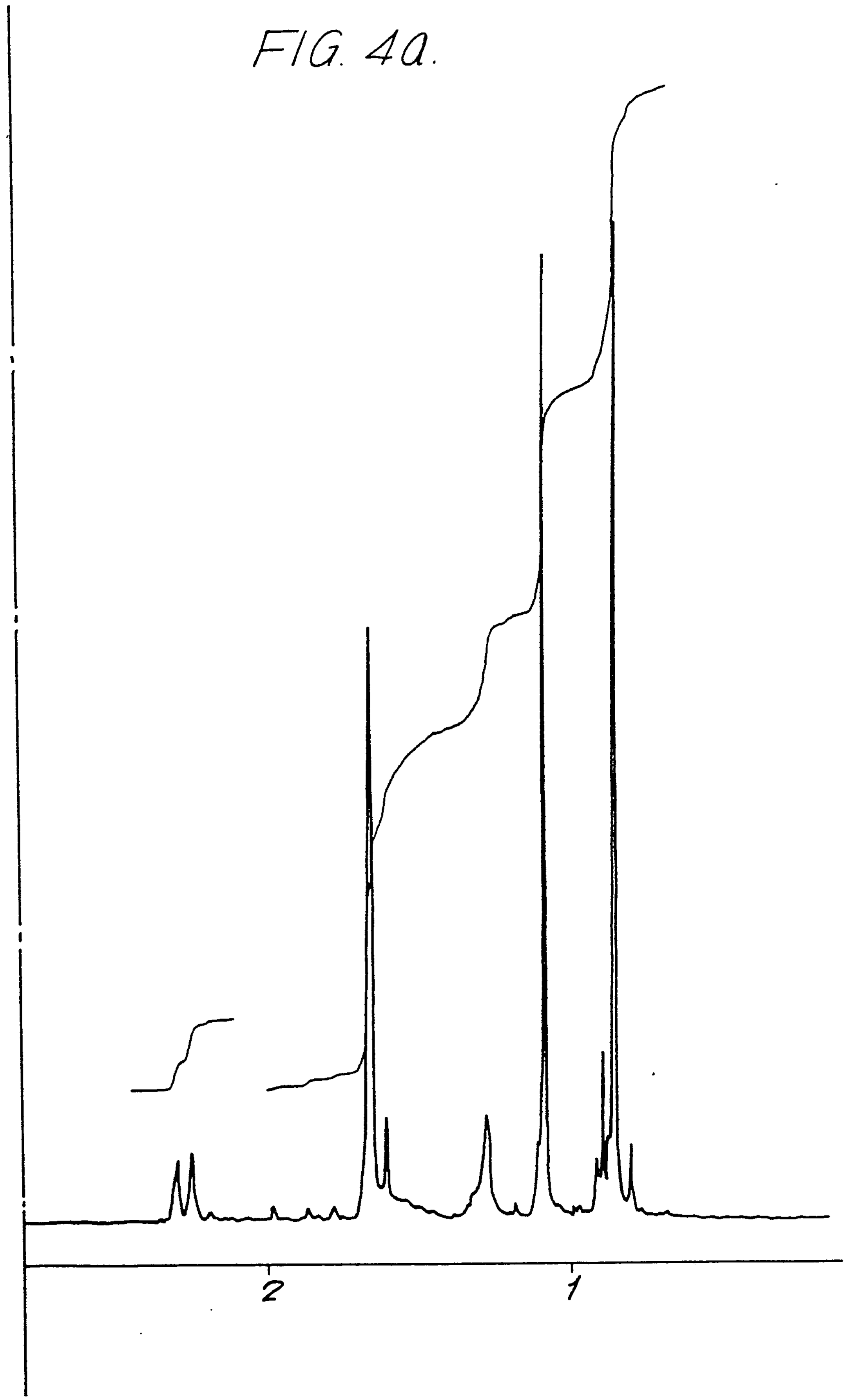

FIG. 4b.

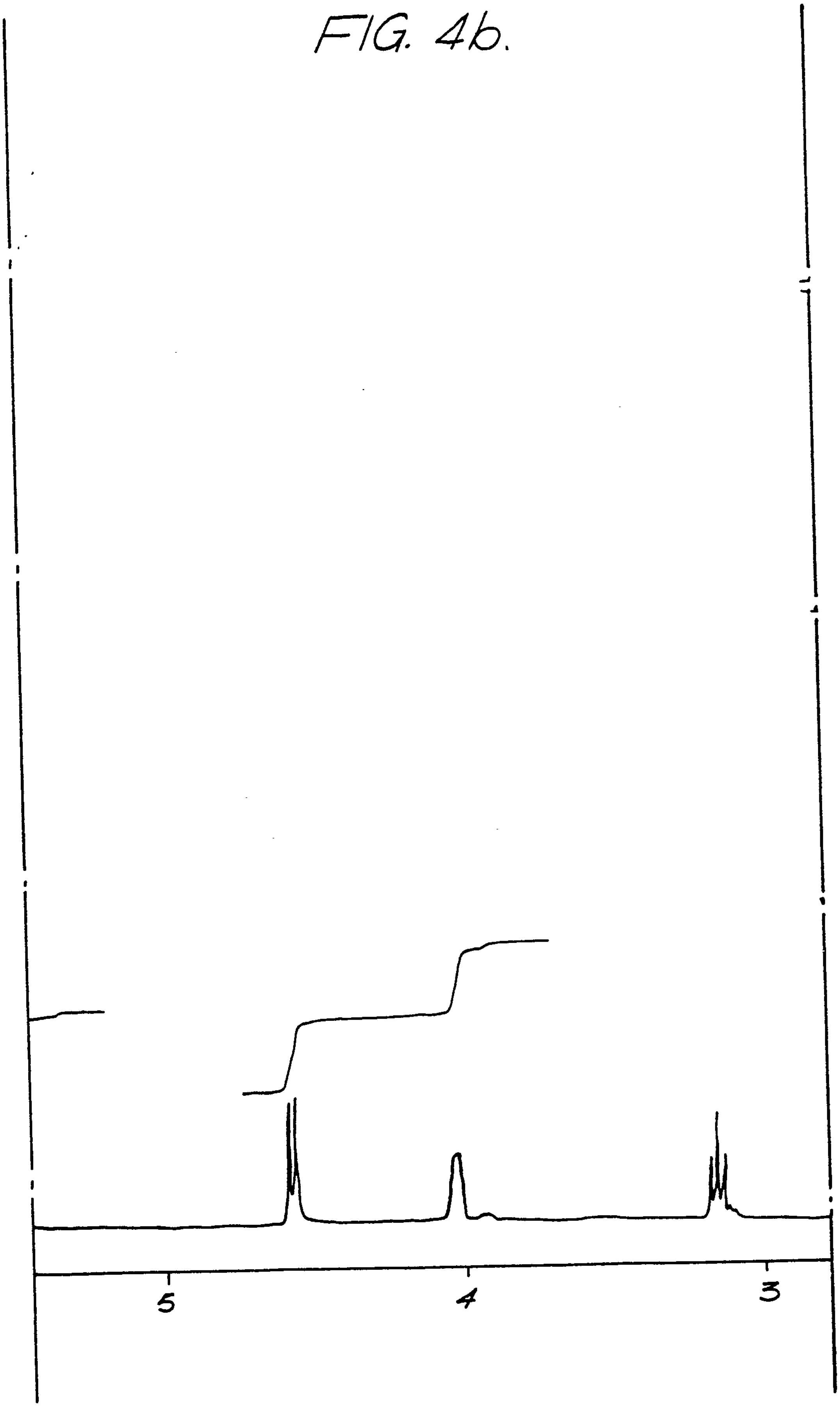

FIG. 4C.

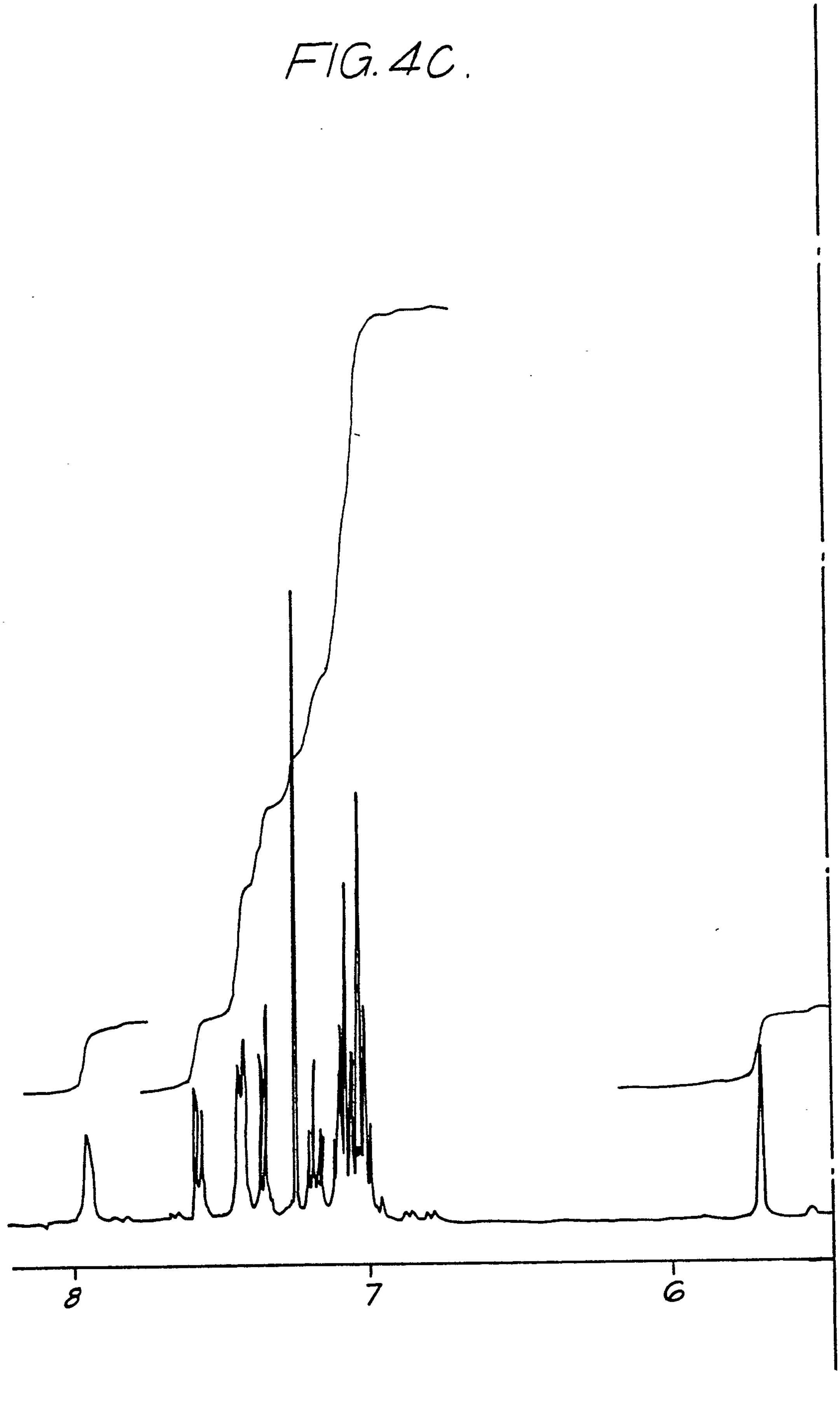

FIG.5.

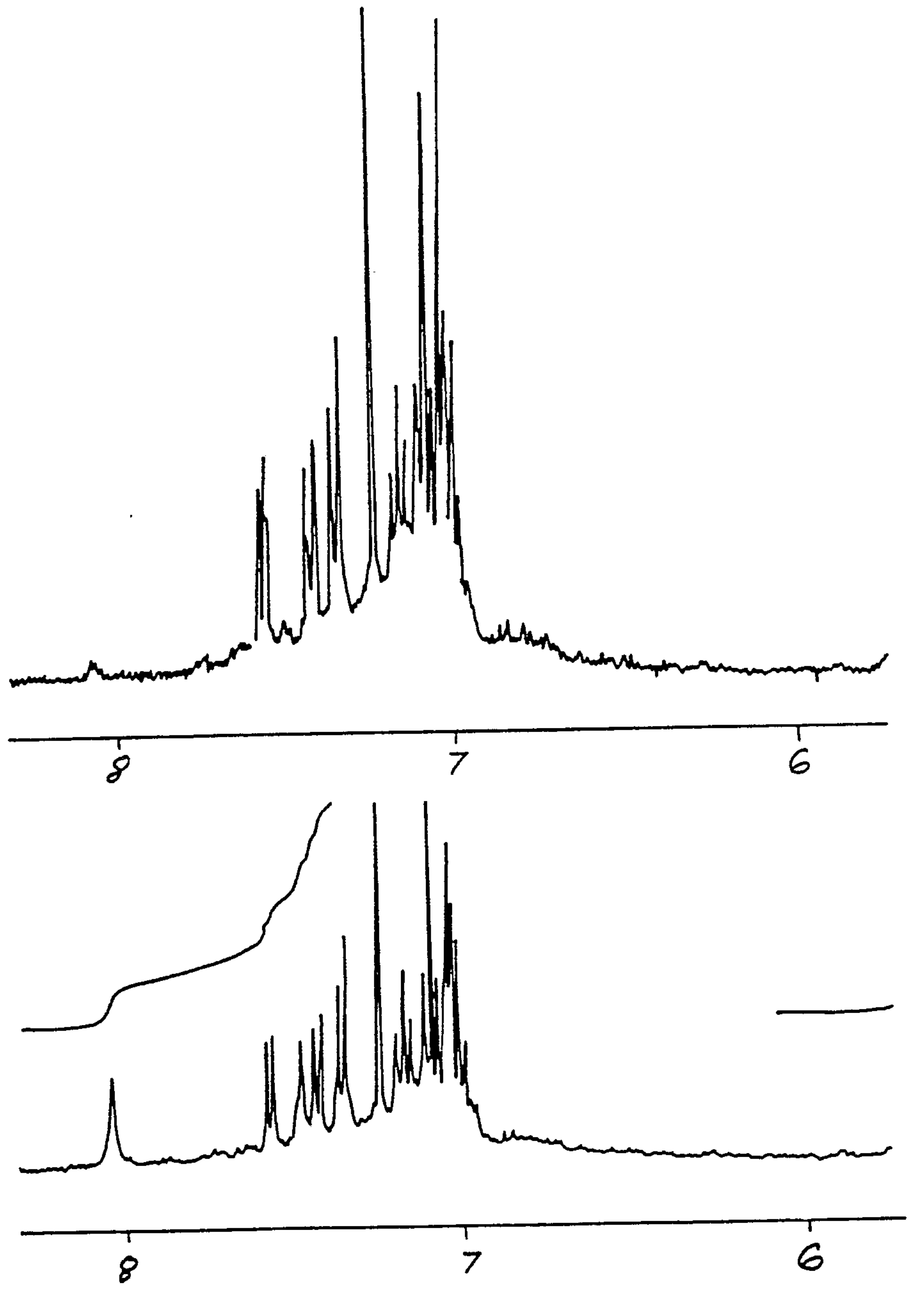

FIG. 6a.

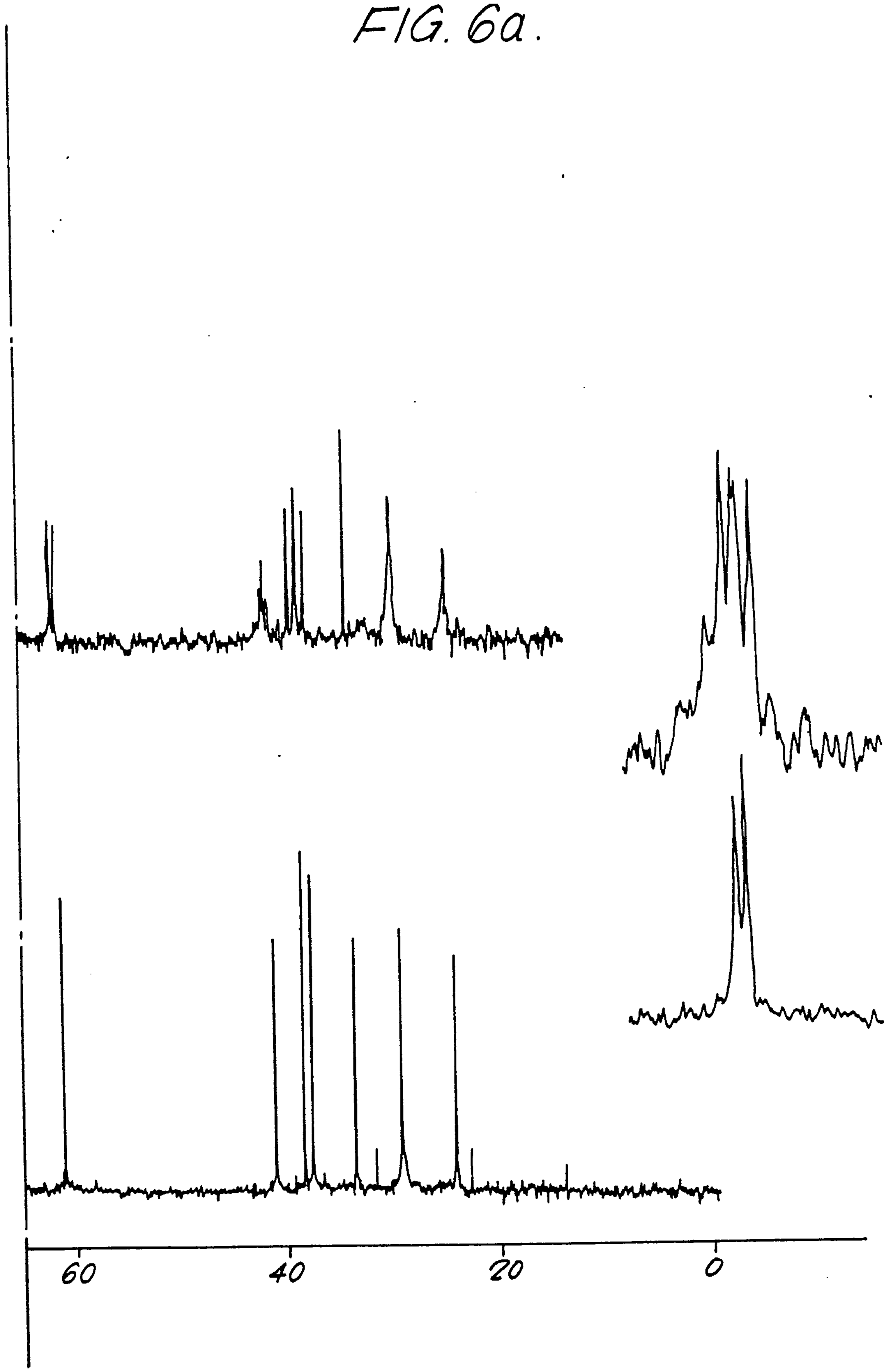

FIG. 6b.

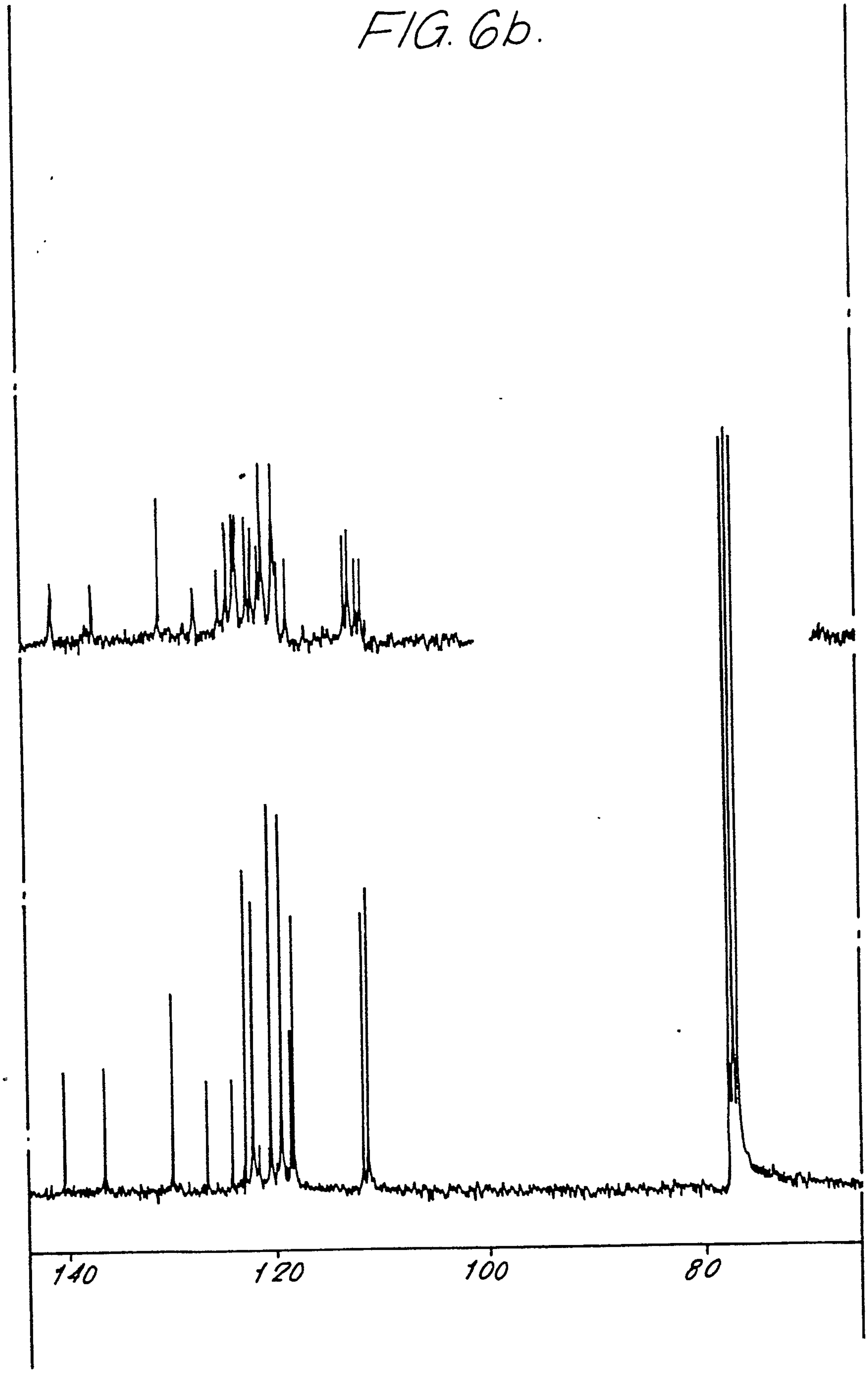

FIG. 6C.

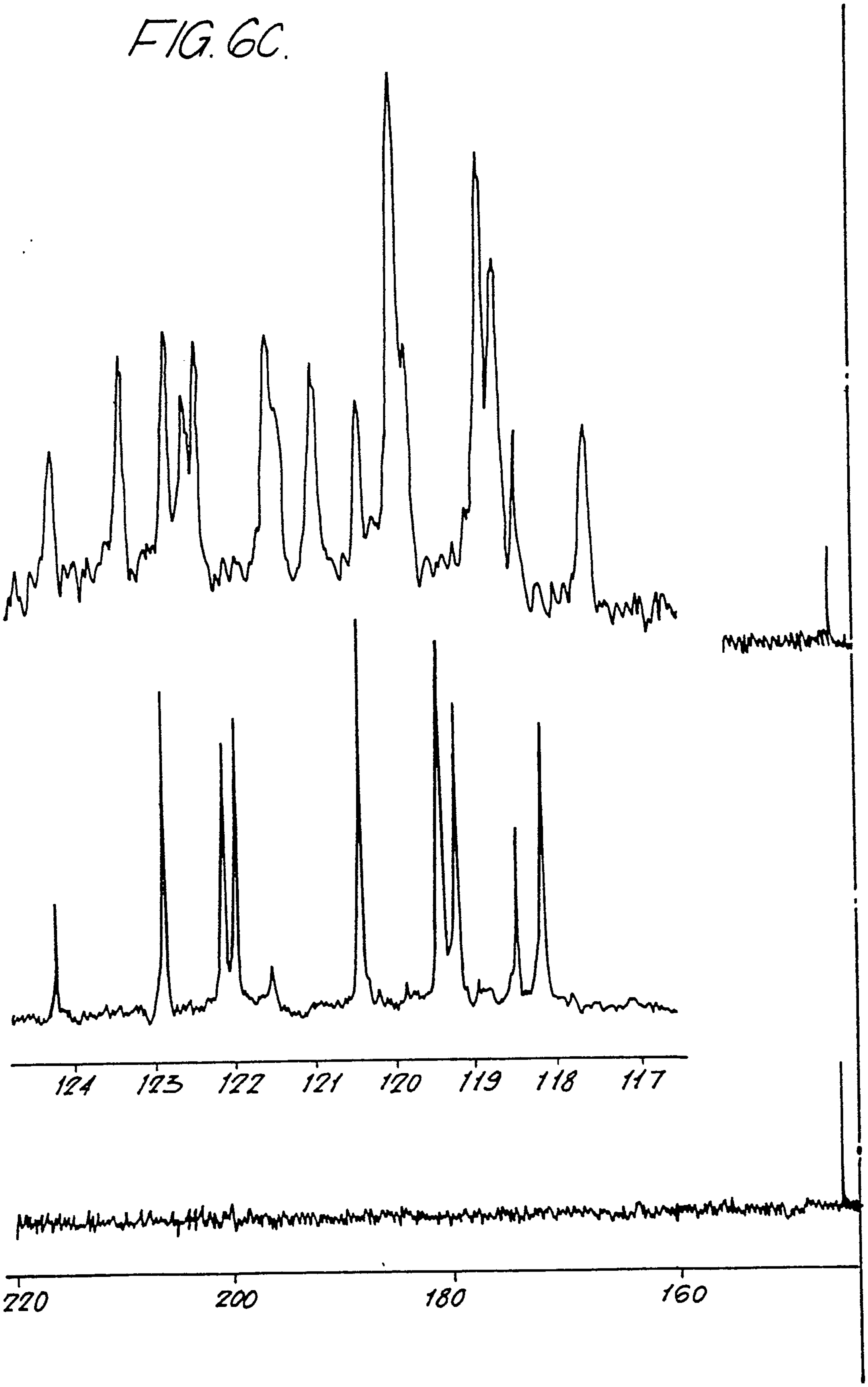

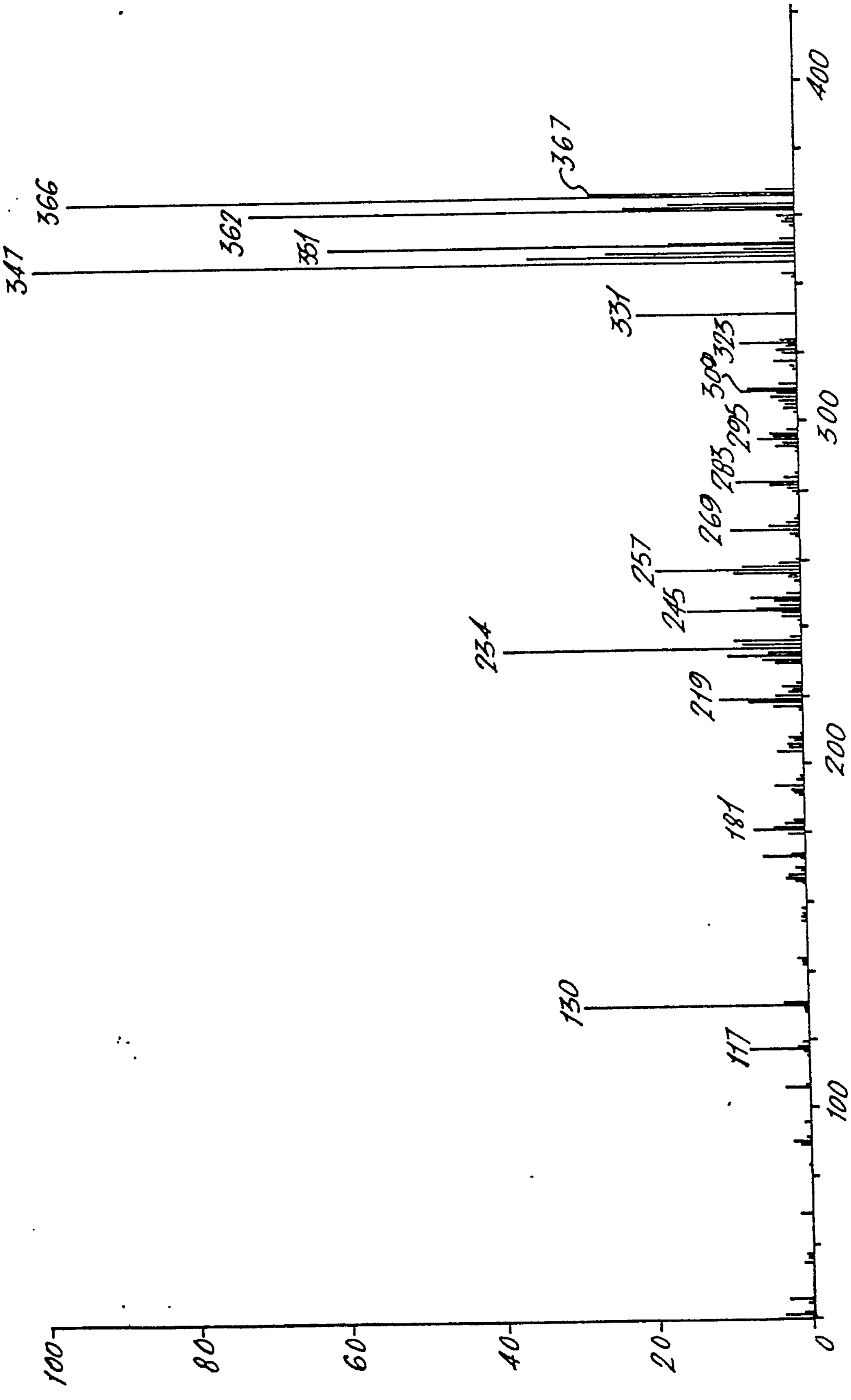
367
366
362
351
341
331
323
308
295
283
269
257
245
234
219
181
130
117
100
200
300
400
0
20
40
60
80
100

FIG. 7.

FIG. 8.

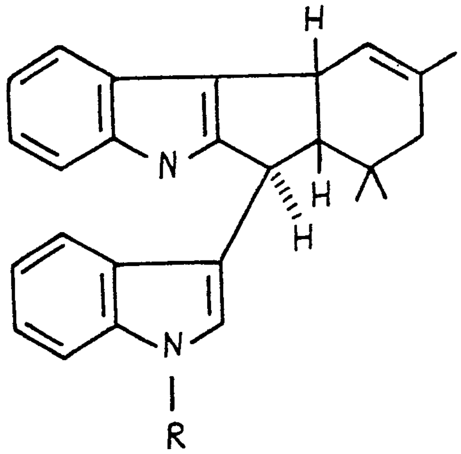

Ia

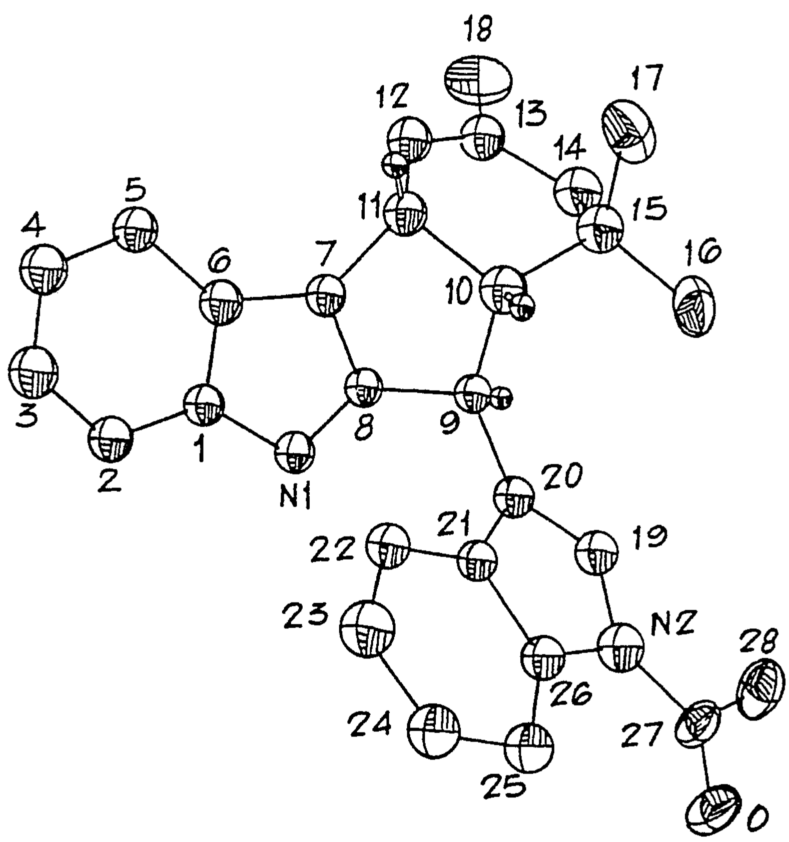

FIG. 9a

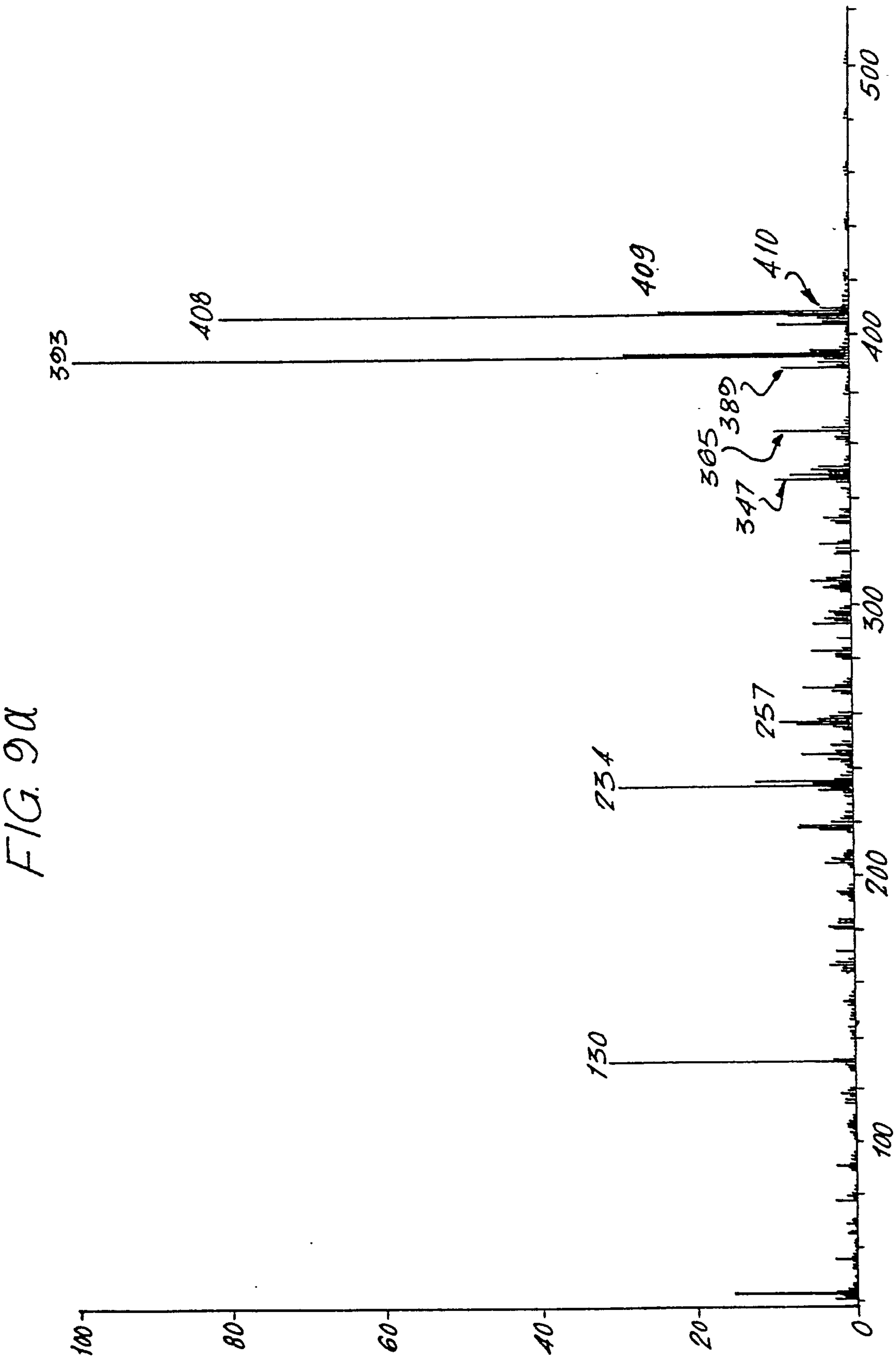
393
408
409
410
389
385
347
257
234
130
0
100
200
300
400
500
20
40
60
80
100

FIG. 9b.

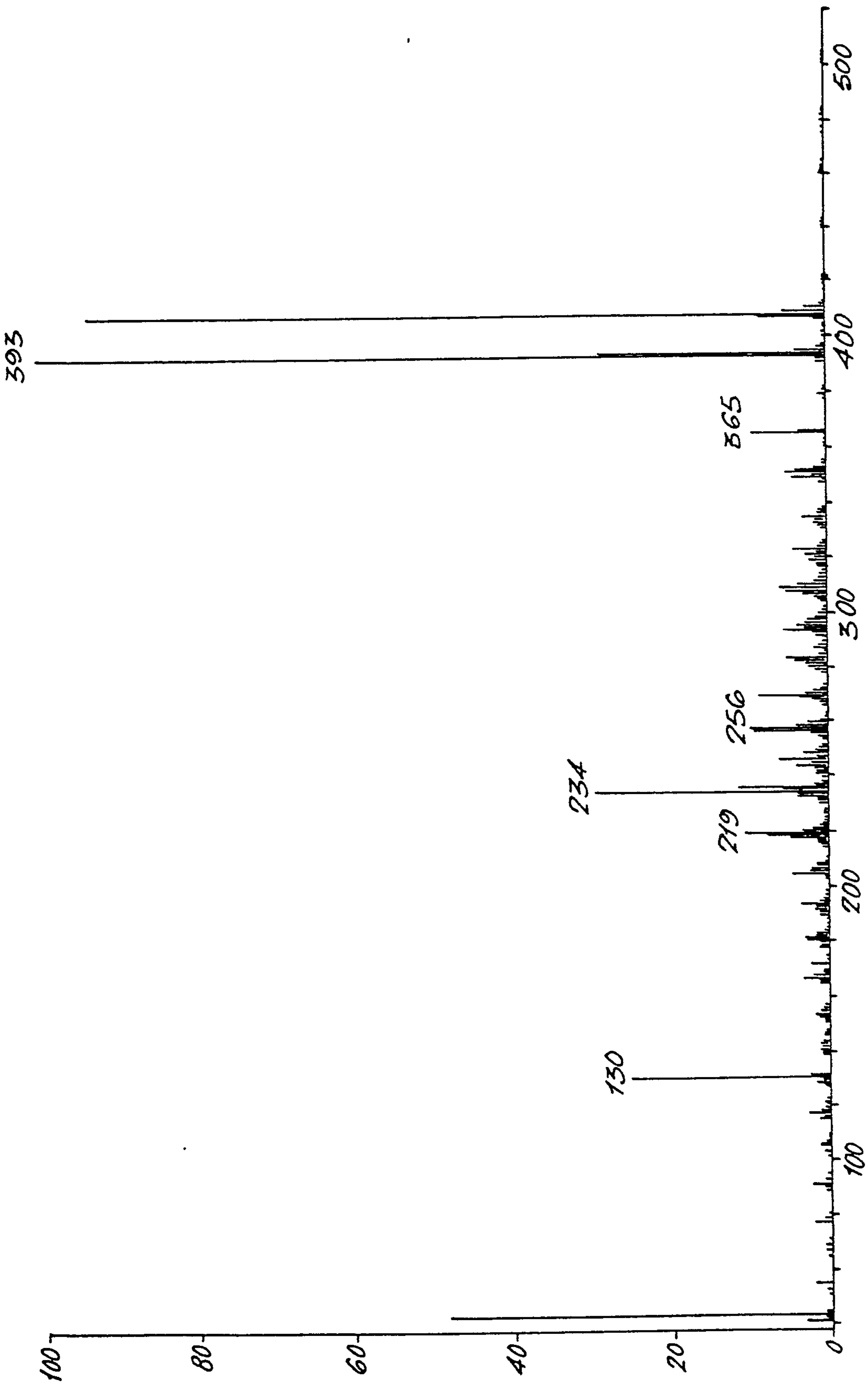

FIG. 10a.

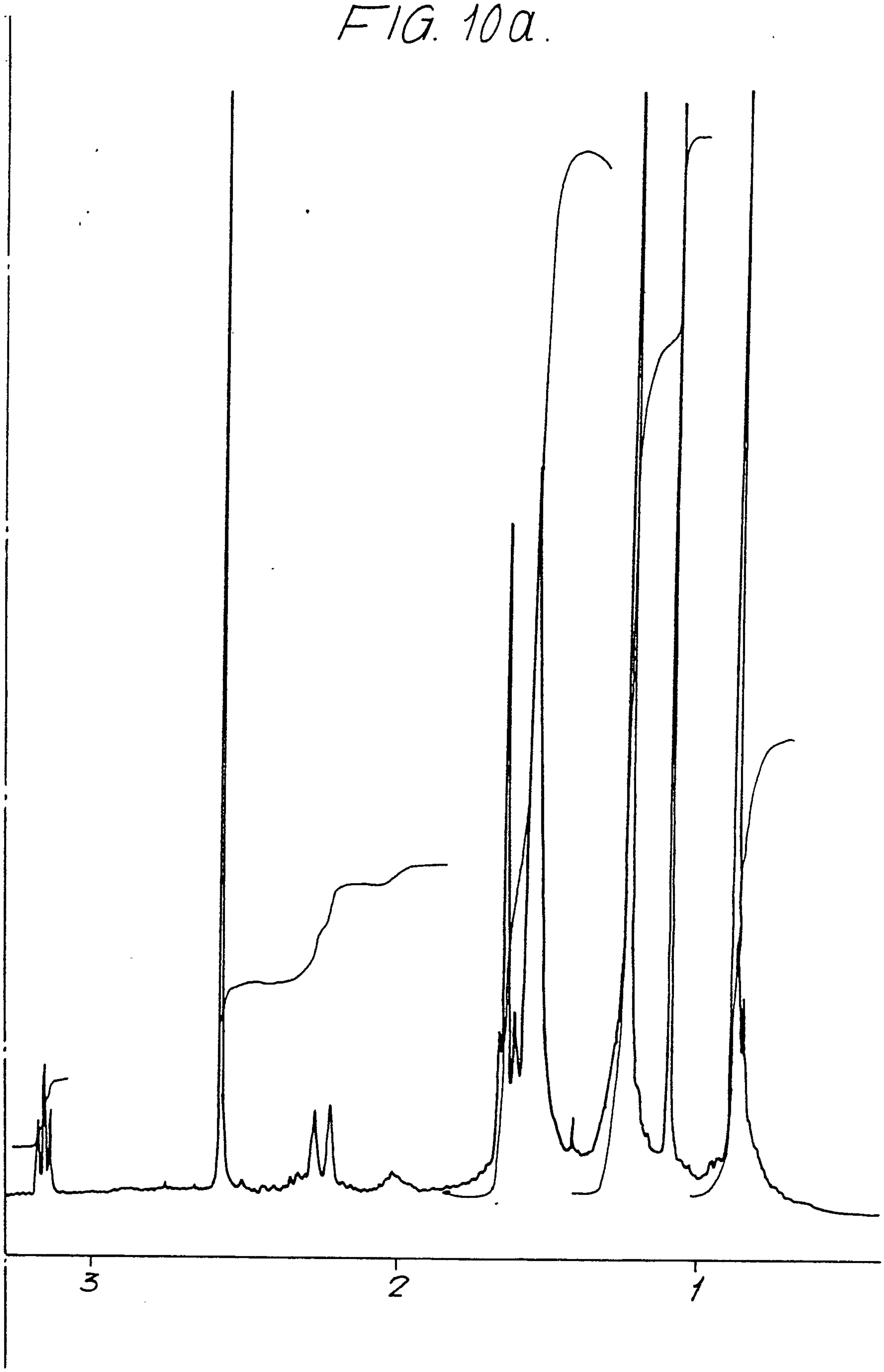

FIG. 10b.

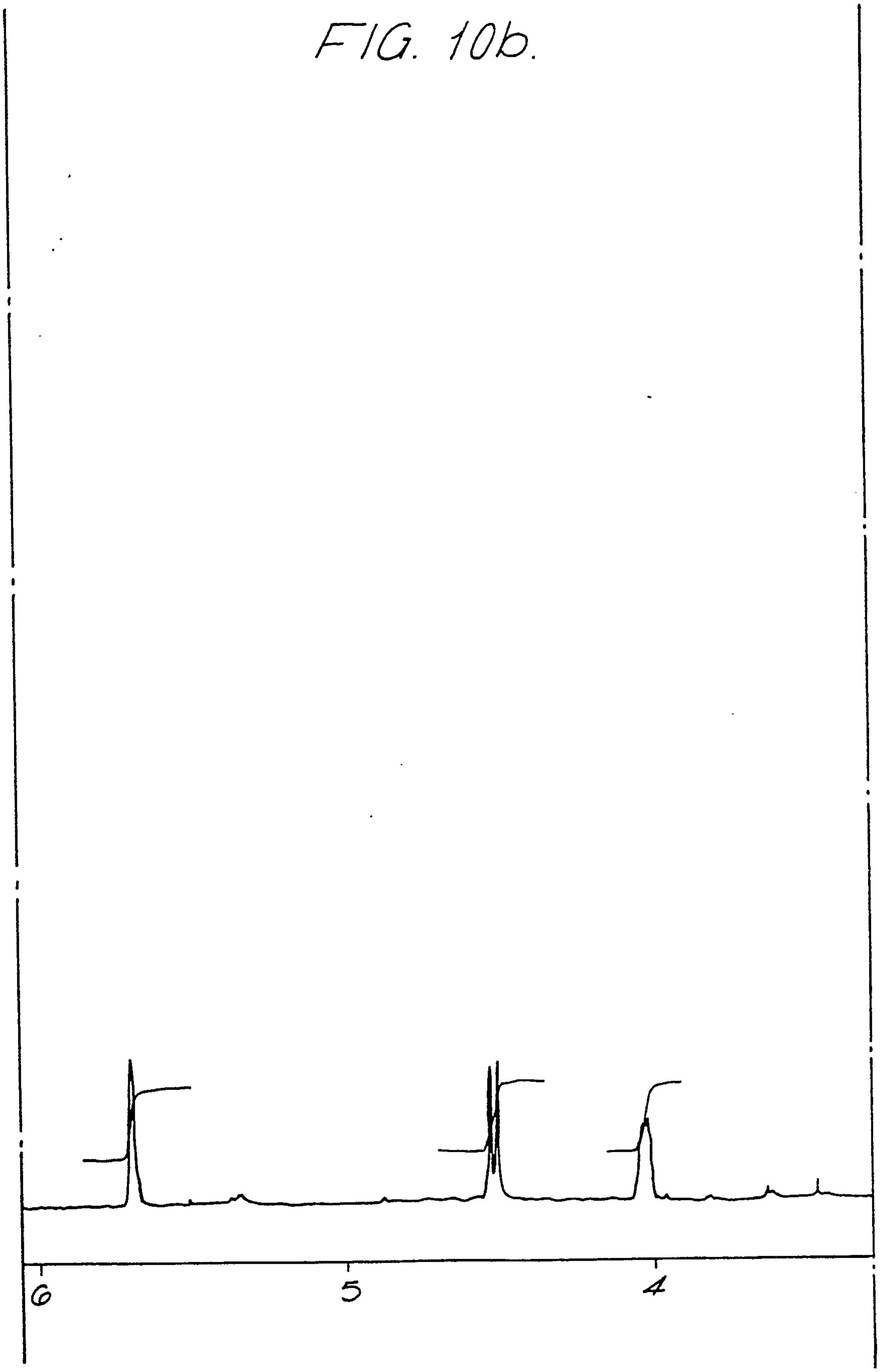

FIG. 10C.